(19) Europäisches Patentamt
European Patent Office
Office européen des brevets

(11) Veröffentlichungsnummer: **0 007 544**
**B1**

# (12) EUROPÄISCHE PATENTSCHRIFT

(45) Veröffentlichungstag der Patentschrift: **02.09.81**

(51) Int. Cl.³: **C 07 F 9/165**, A 01 N 57/16

(21) Anmeldenummer: **79102461.5**

(22) Anmeldetag: **16.07.79**

(54) **Thienyl-dithiophosphorsäureester, Verfahren zu ihrer Herstellung, ihre Verwendung als Schädlingsbekämpfungsmittel, Verfahren zu deren Herstellung und Verfahren zur Bekämpfung von Schädlingen.**

(30) Priorität: **20.07.78 DE 2831936**

(43) Veröffentlichungstag der Anmeldung: **06.02.80 Patentblatt 80/3**

(45) Bekanntmachung des Hinweises auf die Patenterteilung: **02.09.81 Patentblatt 81/35**

(84) Benannte Vertragsstaaten: **AT BE CH DE FR GB IT LU NL SE**

(56) Entgegenhaltungen:
**DE-A-2 111 192**
**DE-A1-2 806 649**
**US-A-3 205 238**
**US-A-3 406 233**

(73) Patentinhaber: **BASF Aktiengesellschaft, Carl-Bosch-Strasse 38, D-6700 Ludwigshafen (DE)**

(72) Erfinder: **Baumann, Annegrit, Dr. Chem., Im Sennteich 26, D-6800 Mannheim 24 (DE)**
Erfinder: **Adolphi, Heinrich, Dr., Kalmitweg 11, D-6703 Limburgerhof (DE)**

**EP 0 007 544 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Thienyl-dithiophosphorsäureester, Verfahren zu ihrer Herstellung, ihre Verwendung als Schädlingsbekämpfungsmittel, Verfahren zu deren Herstellung und Verfahren zur Bekämpfung von Schädlingen

Die vorliegende Erfindung betrifft neue Thienylmethyl-dithiophosphorsäureester, ein Verfahren zu ihrer Herstellung, Schädlingsbekämpfungsmittel, die diese Phosphorsäureester als Wirkstoffe enthalten, ihre Verwendung sowie ein Verfahren zur Herstellung dieser Mittel.

Insektizid wirksame O,O-Dialkyl-S-thienylmethyl-thiophosphorsäureester sind aus der US-PS 3 205 238 und der US-PS 3 406 233 bekannt. Dabei kann der Thienylrest unsubstituiert sein oder bis zu zwei Halogensubstituenten tragen.

Es wurde nun gefunden, daß Thienylmethyl-dithiophosphorsäureester der Formel I

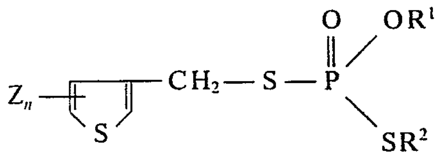

(I)

in der

$R^1$ Methyl oder Äthyl,
$R^2$ unverzweigtes oder verzweigtes Alkyl mit bis zu 5 Kohlenstoffatomen,
Z Halogen und
n 0 bis 3

bedeuten, in ihrer Wirksamkeit den bekannten O,O-Dialkyl-S-thienylmethyl-thiophosphorsäureestern überlegen sind. Die erfindungsgemäßen Verbindungen eignen sich hervorragend zur Bekämpfung von Schädlingen aus den Klassen Insekten, Arachnoidea und Nemathelminthes.

In der Formel I stehen $R^1$ vorzugsweise für Äthyl und $R^2$ für unverzweigtes oder verzweigtes Alkyl mit bis zu 5 Kohlenstoffatomen, beispielsweise für Methyl, Äthyl, Propyl-, Butyl- und Pentylreste, vorzugsweise für Propyl- und Butylreste, insbesondere für n-Propyl. Z bedeutet Halogen, vorzugsweise Chlor oder Brom.

Gegenstand der Erfindung ist weiterhin ein Verfahren zur Herstellung der Thienylmethyl-dithiophosphorsäureester der Formel I, das dadurch gekennzeichnet ist, daß man Thiophenderivate der Formel II

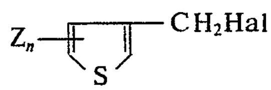

(II)

in der
Z und n die obengenannten Bedeutungen haben und
Hal für Chlor oder Brom steht,
mit Salzen der Formel III

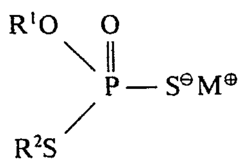

(III)

in der
$R^1$ und $R^2$ die obengenannten Bedeutungen haben und
$M^{\oplus}$ für ein gegebenenfalls substituiertes Ammoniumion steht,
in Gegenwart eines Lösungs- oder Verdünnungsmittels umsetzt.

Geeignete Lösungs- oder Verdünnungsmittel sind beispielsweise aromatische Kohlenwasserstoffe, wie Benzol, Toluol, Xylole, Chlorbenzol; chlorierte oder nitrierte aliphatische Kohlenwasserstoffe, wie Chloroform, 1,2-Dichloräthan, Nitromethan; aliphatische Nitrile, wie Acetonitril, Propionitril; acyclische und cyclische Äther, wie Diäthyläther und Tetrahydrofuran; acyclische und cyclische Ketone, wie Aceton, Methyläthylketon, Cyclohexanon; Amide, wie Dimethylformamid. Auch Wasser kommt als Lösungsmittel in Betracht. Ebenso können Gemische dieser Lösungsmittel verwendet werden.

Die Reaktionstemperatur kann innerhalb eines größeren Bereichs variiert werden. Im allgemeinen arbeitet man zwischen 10 und 100° C, vorzugsweise zwischen 40 und 50° C, soweit nicht der Siedepunkt des Lösungsmittels die Temperatur nach oben begrenzt.

Zur Durchführung des erfindungsgemäßen Verfahrens setzt man die Thiophenderivate der Formel II und die Salze der Formel III im allgemeinen in äquimolaren Mengen oder zweckmäßigerweise die Salze der Formel III in einem 10%igen Überschuß ein. Ein größerer Überschuß bringt keine Vorteile. Im allgemeinen wird das Thiophenderivat der Formel II zusammen mit dem Lösungs- oder

Verdünnungsmittel vorgelegt, dann wird das Salz der Formel III, ebenfalls im Lösungsmittel gelöst oder suspendiert, zugegeben. Nach mehrstündiger Reaktionsdauer wird dann der Reaktionsansatz nach Abziehen des Lösungsmittels nach üblichen Methoden aufgearbeitet. Wenn erforderlich, kann eine chromatographische Reinigung des anfallenden Produktes angeschlossen werden.

Die neuen Verbindungen fallen in Form von Ölen an. Zu ihrer Charakterisierung dienen Brechungsindex und Elementaranalyse.

Die Thiophenderivate der Formel II können durch Reduktion eines Thiophenaldehyds der Formel IV

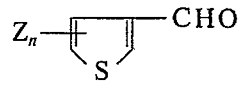

(IV)

in der Z und n die obengenannten Bedeutungen haben, mit einem komplexen Hydrid und durch weitere Umsetzung der so erhaltenen Hydroxymethylverbindung mit einem anorganischen Säurechlorid oder -bromid oder mit konzentrierter Chlorwasserstoff- oder Bromwasserstoffsäure erhalten werden.

Geeignete komplexe Hydride sind Lithiumhydride oder Borhydride, beispielsweise $LiAlH_4$, $LiAl(OCH_3)_2H_2$, $NaBH_4$, $KBH_4$ oder andere Hydride vergleichbarer Reaktivität. Das bevorzugte Reduktionsmittel ist $NaBH_4$, da die Umsetzung auch in wäßrigen Lösungsmitteln durchgeführt werden kann. In der Regel verwendet man äquimolare Mengen an Hydrid, bezogen auf Thiophenaldehyd.

Die Reduktion wird zweckmäßigerweise in einem inerten oder weitgehend inerten Lösungsmittel durchgeführt. Verwendet man $NaBH_4$, wählt man niedere Alkohole, wie Mathanol, Äthanol, Isopropanol oder Isobutanol, oder auch Dialkyläther oder cyclische Äther, wie Diäthyläther, Methyl-tert.-butyläther, Dioxan, Tetrahydrofuran oder Kohlenwasserstoffe als Lösungsmittel. Vorzugsweise wird die Reaktion jedoch in Wasser oder in homogenen oder heterogenen Mischungen von Wasser mit einem der oben angegebenen Solventien durchgeführt, wenn $NaBH_4$ als Reduktionsmittel dient.

Die Reduktion wird bei Temperaturen von $-10°C$ bis $60°C$, vorzugsweise zwischen 0 und $40°C$, durchgeführt.

Die Aufarbeitung des Reaktionsgutes kann in an sich bekannter Weise erfolgen, z. B. durch Zugabe von Essigester, Alkoholen, Wasser oder Mischungen von organischen Solventien mit Wasser. Wurde mit $NaBH_4$ als Reduktionsmittel gearbeitet, wird nur so viel Wasser zugegeben, daß sich das entstandene 4-Hydroxymethyl-thiophen direkt abtrennen läßt. Die Phasentrennung kann durch organische Solventien unterstützt werden.

Zur Herstellung der 4-Chlormethyl- oder 4-Brommethyl-thiophen-Verbindungen der Formel II versetzt man die entsprechenden 4-Hydroxymethyl-thiophene zweckmäßigerweise in einem inerten Lösungsmittel in suspendierter, emulgierter oder gelöster Form mit einem anorganischen Säurechlorid oder Säurebromid, wie $SOCl_2$, $SOBr_2$, $PCl_3$, $PBr_3$ oder $POCl_3$. Gegebenenfalls kann man einen Katalysator, beispielsweise ein tertiäres organisches Amin, zusetzen.

Als inerte Lösungsmittel eignen sich vor allem aliphatische Halogenkohlenwasserstoffe, wie $CH_2Cl_2$, $C_2H_4Cl_2$ oder auch $CHCl_3$, gesättigte oder ungesättigte Kohlenwasserstoffe, wie n-Pentan, n-Hexan, Benzin, aromatische Kohlenwasserstoffe, wie Toluol, Xylole oder Chlorbenzol. Wenn möglich, wird ohne Solvens gearbeitet und ein deutlicher Überschuß des Halogenierungsmittels als Lösungsmittel verwendet.

Nach einer anderen Verfahrensweise können konzentrierte, wäßrige oder nichtwäßrige Brom- oder Chlorwasserstoffsäure verwendet werden. In diesem Fall genügt ein einfaches Ausrühren des 4-Hydroxymethyl-thiophens mit konzentrierter Salzsäure oder Bromwasserstoffsäure bei Raumtemperatur, Verdünnen mit einem inerten Solvens, wie Methylenchlorid, und übliche Isolierung des erhaltenen 4-Halogenmethyl-thiophens.

Die Thiophenaldehyde der Formel IV, in der n 1 bis 3 bedeutet, sind bekannt (Tetrahedron 32, 1403—1406 [1976]) und können durch Halogenieren von Thiophen-3-aldehyd hergestellt werden.

Herstellung von 2,3-Dichlor-4-chlormethyl-thiophen

In einem 2-l-Kolben werden 300 ml Isopropanol, 300 ml Wasser und 200 g 2,3-Dichlor-thiophen-4-aldehyd vorgelegt und innerhalb von 45 Minuten portionsweise mit 16 g Natriumborhydrid versetzt. Die Reaktion verläuft exotherm. Sie wird unter Kühlung bei 35 bis $40°C$ durchgeführt. Anschließend wird die zweiphasige Mischung 30 Minuten bei dieser Temperatur nachgerührt und mit 300 ml Wasser und 500 ml Dichlormethan versetzt. Nach Phasentrennung wird die wäßrige Phase zweimal mit 250 ml Dichlormethan extrahiert. Die organischen Phasen werden mit 250 ml Wasser gewaschen und eingeengt. Der ölige Rückstand wird dann in Cyclohexan aufgenommen; man erhält 182 g kristallines 2,3-Dichlor-4-hydroxymethyl-thiophen, Schmp. 63 bis $65°C$; Ausbeute 87,3% d. Th.

Eine Mischung von 357 g Thionylchlorid und 150 ml Chloroform wird in einem 1-l-Kolben vorgelegt, auf $10°C$ abgekühlt und innerhalb 35 Minuten bei 10 bis $15°C$ mit einer Lösung von 238 g

2,3-Dichlor-4-hydroxymethyl-thiophen versetzt. Nach Abklingen der exothermen Reaktion wird die Lösung eine Stunde bei 30° C nachgerührt, unter vermindertem Druck eingeengt und nach Zugabe von wenig Triäthylamin destillativ bei 66 bis 69° C/0,2 mbar gereinigt. Die Ausbeute beträgt 192 g, entsprechend 70% d. Th.

Analog können folgende Verbindungen hergestellt werden:

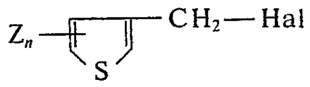

| Substituenten am Thiophen-ring ($Z_n$) | Hal | Kp/Fp |
|---|---|---|
| – | Cl | 46°C/0,8 mbar |
| 2-Brom | Cl | 63–68°C/0,27 mbar |
| 2,3-Dibrom | Cl | 141–146°C |
| 2,5-Dibrom | Cl | 74–76°C/0,07 mbar |
| 2,3,5-Tribrom | Cl | 51–53°C |
| 2-Brom | Br | 88–90°C/0,93 mbar |
| 2-Chlor | Cl | 51–53°C/0,33 mbar |
| 2,3-Dichlor | Br | 58–60°C/0,13 mbar |
| 2-Chlor | Br | 61–62°C/0,53 mbar |

Die folgenden Beispiele erläutern die Herstellung der erfindungsgemäßen Thienylmethyl-thiophosphorsäureester der Formel I.

## Beispiel 1

0,05 Mol 2,3-Dichlor-4-chlormethylthiophen werden in 100 ml Acetonitril vorgelegt und mit 0,05 Mol O-Äthyl-S-isobutyldithiophosphorsaurem Dimethylammoniumsalz gelöst, in 100 ml Acetonitril, versetzt. Nach 2 Stunden Rühren bei Zimmertemperatur wird noch 2 Stunden lang auf 50° C erwärmt, dann wird das Lösungsmittel entfernt, der Rückstand mit Äther aufgenommen, die Ätherphase mit Wasser ausgeschüttelt, getrocknet und eingeengt. Man erhält 13,7 g O-Äthyl-S-isobutyl-S-(2,3-dichlor-thienyl-4-methyl)-dithiophosphat, entsprechend einer Ausbeute von 72% d. Th. Die leicht verunreinigte Probe wird über eine Säule mit Kieselgel gegeben; $n_D^{25}$ = 1,5712.

Analyse:

| | | | | | |
|---|---|---|---|---|---|
| theor. | C 34,8 | H 4,5 | S 25,3 | Cl 18,7 | P 8,2 |
| gef. | C 35,0 | H 4,5 | S 25,1 | Cl 19,1 | P 7,7 |

## Beispiel 2

0,035 Mol 2,5-Dibrom-3-chlormethylthiophen und 0,037 Mol O-Äthyl-S-n-propyldithiophosphorsaurem Dimethylammoniumsalz, gelöst in 200 ml Tetrahydrofuran, werden analog Beispiel 1 umgesetzt. Eine Reinigung des Reaktionsproduktes ist nicht erforderlich. Man erhält 12,9 g O-Äthyl-S-n-propyl-S-(2,5-dibrom-thienyl-4-methyl)-dithiophosphat, entsprechend einer Ausbeute von 82% d. Th.; $n_D^{25}$ = 1,5987.

Analyse:

| | | | | | |
|---|---|---|---|---|---|
| theor. | C 26,4 | H 3,3 | S 21,2 | Br 35,2 | P 6,8 |
| gef. | C 26,9 | H 3,4 | S 21,2 | Br 36,0 | P 6,6 |

Analog können folgende Verbindungen synthetisiert werden:

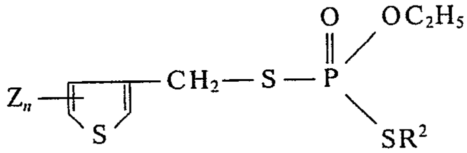

| Nr. | $R^3 = Z_n$ 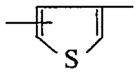 | $R^2$ | $n_D^{25}$ |
|---|---|---|---|
| 3 | 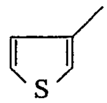 | sec-$C_4H_9$ | 1,5591 |
| 4 | 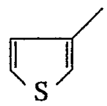 | i-$C_4H_9$ | 1,5596 |
| 5 | 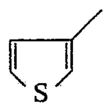 | i-$C_3H_7$ | 1,5661 |
| 6 | 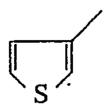 | $CH_3$ | 1,5818 |
| 7 | 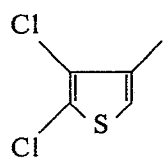 | n-$C_3H_7$ | 1,5703 |
| 8 | 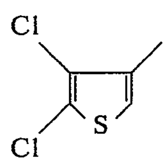 | sec-$C_4H_9$ | ./. |
| 9 | 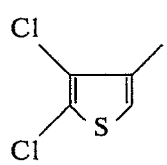 | i-$C_4H_9$ | 1,5712 |
| 10 | 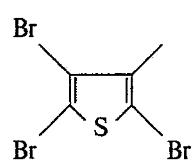 | i-$C_4H_9$ | 1,6063 |
| 11 | 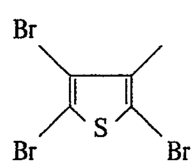 | sec-$C_4H_9$ | 1,6067 |
| 12 | 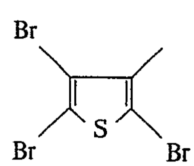 | n-$C_3H_7$ | 1,6121 |

(Fortsetzung)

| Nr. | $R^3 = Z_n$ 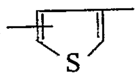 | $R^2$ | $n_D^{25}$ |
|---|---|---|---|
| 13 | 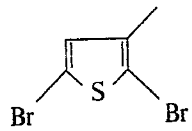 | sec-$C_4H_9$ | 1,5934 |
| 14 | 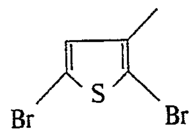 | i-$C_4H_9$ | 1,5883 |
| 15 | 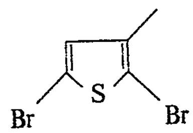 | n-$C_3H_7$ | 1,5987 |
| 16 | 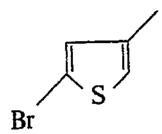 | i-$C_4H_9$ | 1,5724 |
| 17 | 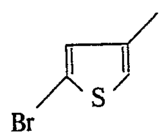 | n-$C_3H_7$ | 1,5859 |
| 18 | 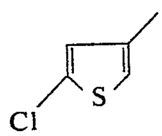 | sec-$C_4H_9$ | 1,5632 |
| 19 | 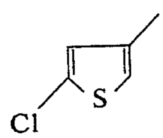 | i-$C_4H_9$ | 1,5643 |
| 20 | 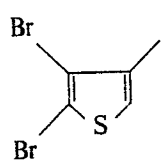 | i-$C_3H_7$ | 1,6018 |
| 21 | 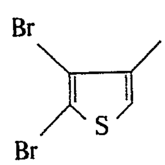 | i-$C_4H_9$ | 1,5888 |
| 22 | 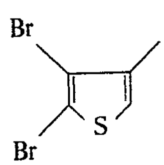 | sec-$C_4H_9$ | 1,5968 |

Die erfindungsgemäßen Verbindungen sind geeignet, um schädliche oder lästige Gliedertiere aus der Klasse der Insekten, der Arachnoidea und der Nemathelminthes wirksam zu bekämpfen.

Zu den schädlichen Insekten gehören aus der Ordnung der Schmetterlinge (Lepidoptera), beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);
aus der Ordnung der Käfer (Coleoptera), beispielsweise Blitophaga undata (Schwarzer Rübenaaskäfer), Melanotus communis (Drahtwurm), Limonius californicus (Drahtwurm), Agriotes lineatus (Saatschnellkäfer), Agricotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varicestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefruchter Wandgärtner);
aus der Ordnung der Zweiflügler (Diptera), beispielsweise Mayetiola destructor (Hessenfliege), Dasyneura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege);
aus der Ordnung der Hautflügler (Hymenoptera), beispielsweise Athalia rosae (Rübsenblattwespe), Hoplocampa minuta (Pflaumensägewespe), Monomorium pharaonis (Pharaoameise), Solenopsis geminata (Feuerameise), atta sexdens (Blattschneiderameise);
aus der Ordnung der Wanzen (Heteroptera), beispielsweise Nezara viridula (Grüne Reiswanze), Eurygaster integriceps (Asiatische Getreidewanze), Blissus leucopterus (Chinch bug.), Dysdercus cingulatus (Kapok-Wanze), Dysdercus intermedius (Baumwollwanze), Piesma quadrata (Rübenwanze), Lygus pratensis (Gemeine Wiesenwanze);
aus der Ordnung der Pflanzensauger (Homoptera), beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera), beispielsweise Reticulitermes lucifugus.

Zur Klasse der Arachnoidea gehören Milben und Zecken (Acarina), beispielsweise Ixodes ricinus (Holzblock), Ornithodorus moubata, Amblyomma americanum, Dermacentor silvarum, Boophilus

microplus, Tetranychus telarius, Tetranychus atlanticus, Tetranychus pacificus, Paratetranychus pilosus, Bryobia praetiosa.

Zur Klasse der Nemathelminthes zählen beispielsweise Wurzelgallennematoden, z. B. Meloidogyne incognita, Meloidogyne hapla, Meloidogyne javanica, Zysten bildende Nematoden, z. B. Heterodera rostochiensis, Heterodera schachtii, Heterodera avenae, Heterodera glycines, Heterodera trifolii, Stock- und Blattälchen, z. B. Ditylenchus dipsaci, Ditylenchus destructor, Pratylenchus neglectus, Pratylenchus penetrans, Partylenchus goodeyi, Paratylenchus curvitatus sowie Tylenchorhynchus dubius, Tylenchorhynchus claytone, Rotylenchus robustus, Heliocotylenchus multicinctus, Radopholus similis, Belonolaimus longicaudatus, Longidorus elongatus, Trichodorus primitivus.

Die erfindungsgemäßen Verbindungen können mit Erfolg im Pflanzenschutz sowie auf dem Hygiene-, Vorratsschutz- und Veterinärsektor als Schädlingsbekämpfungsmittel eingesetzt werden.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenon, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit über 95 Gew.-% Wirkstoff oder sogar den 100%igen Wirkstoff allein auszubringen.

Beispiele für Formulierungen sind:

I. 3 Gewichtsteile O-Äthyl-S-isobutyl-S-(2,3-dichlor-thienyl-4-methyl)-dithiophosphat werden mit 97 Gewichtsteilen feinteiligem Kaolin innig vermischt. Man erhält auf diese Weise ein Stäubemittel, das 3 Gew.-% des Wirkstoffs enthält.

II. 30 Gewichtsteile O-Äthyl-S-n-propyl-S-(2,5-dibrom-thienyl-4-methyl)-dithiophosphat werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 20 Gewichtsteile O-Äthyl-S-sec.-butyl-S-(2,5-dibrom-thienyl-4-methyl)-dithiophosphat werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile O-Methyl-S-isobutyl-S-(2-chlor-thienyl-4-methyl)-dithiophosphat werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Zu den Einzelwirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan, 1,3-Dichlorpropen, 1,3-Dichlorpropben + 1,2-Dichlorpropan,
1,2-Dibrom-äthan, 2-sec.-Butyl-phenyl-N-methylcarbamat,
o-Chlorphenyl-N-methylcarbamat, 3-Isopropyl-5-methylphenyl-N-methylcarbamat,
o-Isopropoxyphenyl-N-methylcarbamat,
3,5-Dimethyl-4-methylmercapto-phenyl-N-methylcarbamat,
4-Dimethylamino-3,5-xylyl-N-methylcarbamat,
2-(1,3-Dioxolan-2-yl)-phenyl-N-methylcarbamat, 1-Naphthal-N-methylcarbamat,
2,3-Dihydro-2,2-dimethyl-benzofuran-7-yl-N-methylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)-propionaldehyd-O-(methylcarbamoyl)-oxim,
S-Methyl-N-[(methylcarbamoyl)-oxy]-thio-acetimidat,
Methyl-N',N'-dimethyl-N-[(methylcarbamoyl)oxy]-1-thiooxamidat,
N-(2-Methyl-4-chlor-phenyl)-N',N'-dimethylformamidin,
Tetrachlorthiophen, O,O-Dimethyl-O-(p-nitrophenyl)-phosphorthioat,
O,O-Diäthyl-O-(p-nitrophenyl)-phosphorthioat,
O-Äthyl-O-(p-nitrophenyl)-phenyl-phosphonothioat,
O,O-Dimethyl-O-(3-methyl-4-nitrophenyl)-phosphorthioat,
O,O-Diäthyl-O-(2,4-dichlorphenyl)-phosphorthioat,
O-Äthyl-O-(2,4-dichlorphenyl)-phenyl-phosphonothioat,
O,O-Dimethyl-O-(2,4,5-trichlorphenyl)-phosphorthioat,
O-Äthyl-O-(2,4,5-trichlorphenyl)-äthyl-phosphonothioat,
O,O-Dimethyl-O-(4-brom-2,5-dichlorphenyl)-phosphorthioat,
O,O-Dimethyl-O-(2,5-dichlor-4-jodphenyl)-phosphorthioat,
O,O-Dimethyl-O-(3-methyl-4-methylthiophenyl)-phosphorthioat,
O-Äthyl-O-(3-methyl-4-methylthiophenyl)-isopropyl-phosphoramidat,
O,O-Diäthyl-O-[p-(methylsulfinyl)phenyl]-phosphorthioat,
O-Äthyl-S-phenyl-äthyl-phosphordithioat,
O,O-Diäthyl-[2-chlor-1-(2,4-dichorphenyl)-vinyl]-phosphat,
O,O-Dimethyl-[-2-chlor-1-(2,4,5-trichlorphenyl)]-vinyl-phosphat,
O,O-Dimethyl-S-(1-phenyl)-äthylacetat-phosphordithioat,
Bis-(dimethylamino)-fluorphosphinoxid, Octamethyl-pyrophosphoramid,
O,O,O,O-Tetraäthyldithio-pyrophosphat,
S-Chlormethyl-O,O-diäthyl-phosphordithioat, O-Äthyl-S,S-dipropyl-phosphordithioat,
O,O-Dimethyl-O-2,2-dichlorvinyl-phosphat,
O,O-Dimethyl-1,2-dibrom-2,2-dichloräthylphosphat,
O,O-Dimethyl-2,2,2-trichlor-1-hydroxy-äthylphosphonat,
O,O-Dimethyl-S-[1,2-biscarbäthoxy-äthyl-(1)]-phosphordithioat,
O,O-Dimethyl-O-(1-methyl-2-carbmethoxy-vinyl)-phosphat,
O,O-Dimethyl-S-(N-methyl-carbamoyl-methyl)-phosphordithioat,
O,O-Dimethyl-S-(N-methyl-carbamoyl-methyl)-phosphorthioate,

O,O-Dimethyl-S-(N-methoxyäthyl-carbamoyl-methyl)-phosphordithioat,
O,O-Dimethyl-S-(N-formyl-N-methyl-carbamoylmethyl-phosphordithioat,
O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diäthylcarbamoyl)-vinyl]-phosphat,
O,O-Diäthyl-S-(äthylthio-methyl)-phosphordithioat,
O,O-Diäthyl-S-[(p-Chlorphenylthio)-methyl]-phosphordithioat,
O,O-Dimethyl-S-(2-äthylthioäthyl)-phosphorthioat,
O,O-Dimethyl-S-(2-äthylthioäthyl)-phosphordithioat,
O,O-Dimethyl-S-(2-äthylsulphinyl-äthyl)-phosphorthioat,
O,O-Diäthyl-S-(2-äthylthio-äthyl)-phosphordithioat,
O,O-Dimethyl-S-(2-äthylsulphinyl-äthyl)-phosphorthioat,
O,O-Diäthylthiophosphoryliminophenyl-acetonitril,
O,O-Diäthyl-S-(2-chlor-1-phthalimidoäthyl)-phosphordithioat,
O,O-Diäthyl-S-[6-chlor-benzoxazolon-(2)-yl(3)]-methyldithiophosphat,
O,O-Dimethyl-S-[2-methoxy-1,3,4-thiodiazol-5-onyl(4)-methyl]-phosphordithioat,
O,O-Diäthyl-O-[3,5,6-trichlor-pyridyl-(2)]-phosphorthioat,
O,O-Diäthyl-O-(2-pyrazinyl)-phosphorthioat,
O,O-Diäthyl-O-[2-isopropyl-4-methyl-pyrimidinyl(6)]-phosphorthioat,
O,O-Diäthyl-O-[2-(diäthylamino)-6-methyl-4-pyrimidinyl]-thionophosphat,
O,O-Dimethyl-S-(4-oxo-1,2,3-benzotriazin-3-yl-methyl)-phosphordithioat,
O,O-Dimethyl-S-[(4,6-diamino-1,3,5-triazin-2-yl)-methyl]-phosphordithioat,
O,O-Diäthyl-(1-phenyl-1,2,4-triazol-3-yl)-thionophosphat,
O,S-Dimethyl-phosphor-amido-thioat,
O,S-Dimethyl-N-acetyl-phosphoramidothioat,
γ-Hexachlorcyclohexan, 1,1-Di-(p-methoxyphenyl)-2,2,2-trichlor-äthan,
6,7,8,9,10,10-Hexachloro-1,5,5a,6,9,9a-hexahydro-6,9-methano-2,4,3-benzodioxa-thiepin-3-oxid,
Pyrethrine,
DL-2-Allyl-3-methyl-cyclopenten-(2)-on-(1)-yl-(4)-DL-cis,trans-chrysanthemat,
5-Benzyl-furyl-(3)-methyl-DL-cis,trans-chrysanthemat,
3-Phenoxybenzyl(±)-cis,trans-2,2-dimethyl-3-(2,2-dichlorvinyl)-cyclopropancarboxylat,
α-Cyano-3-phenoxybenzyl(±)-cis,trans-2,2-dimethyl-3-(2,2-dichlorvinyl)-cyclopropancarboxylat,
(s)-α-Cyano-3-phenoxybenzyl-cis(1R,3R)-2,2-dimethyl-3-(2,2-dibromvinyl)-cyclopropancarboxylat,
3,4,5,6-Tetrahydrophthalimidoäthyl-DL-cis,trans-chrysanthemat,
2-Methyl-5-(2-propinyl)-3-furnylmethyl-chrysanthemat,
α-Cyano-3-phenoxybenzyl-α-isopropyl-4-chlorphenylacetat.

Die folgenden Beispiele belegen die biologische Wirkung. Vergleichsmittel sind das aus der DE-PS 819 998 bekannte O,O-Dimethyl-S-(N-methyl-carbamoylmethyl)-phosphordithioat (I) und die aus der US-PS 3 205 238 bekannten Wirkstoffe O,O-Diäthyl-S-(thienyl-4-methyl)-dithiophosphat (II) und O,O-Diäthyl-S-(2,3-dichlor-thienyl-4-methyl)-dithiophosphat (III). Die Numerierung der erfindungsgemäßen Wirkstoffe entspricht den Herstellungsbeispielen und der tabellarischen Aufzählung.

## Beispiel A

### Wirkung gegen Spinnmilben (Tetranychus telarius)

Getopfte Buschbohnen, die das zweite Folgeblattpaar entwickelt haben und einen starken Befall durch die Spinnmilbe Tetranychus telarius aufweisen, werden in der Spritzkabine allseitig mit der wäßrigen Wirkstoffaufbereitung tropfnaß gespritzt. Anschließend stehen die Pflanzen unter Gewächshausbedingungen bei 22 bis 24° C. Nach 6 Tagen wird die Wirkung beurteilt.

| Wirkstoff Nr. | Mortalitätsrate [%] bei Wirkstoffkonzentrationen von | | | |
|---|---|---|---|---|
| | 0,1% | 0,05% | 0,02% | 0,01% |
| 1 | | 100 | 100 | |
| 2 | | 100 | | |
| 3 | | 100 | | |
| 4 | | 100 | | |
| 8 | | 100 | 100 | 100 |
| 10 | | 100 | | |
| 12 | | 100 | | |
| 13 | | 100 | Grenze der Wirkung | |
| 14 | | 100 | 100 | |
| I | | Grenze der Wirkung | | |
| II | 80 | unwirksam | | |
| III | unwirksam | | | |

Beispiel B

Kontaktwirkung auf Moskito-Larven (Aedes aegypti)

200 ml Leitungswasser werden mit der Wirkstoffaufbereitung versetzt und darauf mit 30 bis 40 Moskito-Larven im 4. Larvenstadium besetzt.
Die Versuchstemperatur beträgt 20° C. Nach 24 Stunden wird die Wirkung ermittelt.

| Wirkstoff Nr. | Mortalitätsrate [%] bei Wirkstoffkonzentrationen von | | | |
|---|---|---|---|---|
| | 1 ppm | 0,5 ppm | 0,25 ppm | 0,1 ppm |
| 2 | 100 | 100 | 100 | |
| 8 | 100 | 100 | 100 | |
| 10 | 100 | 100 | 90 | |
| 11 | 100 | 100 | 100 | 80 |
| 12 | 100 | 100 | 90 | |
| 13 | 100 | 100 | 100 | 100 |
| 14 | 100 | 100 | 100 | |
| I | ca. 20 | | | |
| II | unwirksam | | | |
| III | unwirksam | | | |

## Beispiel C

### Zuchtversuch mit Stubenfliegen-Larven (Musca domestica)

50 g eines Nährbodens aus Bäckerhefe, Trockenmilch, Wasser und Agar werden in warmem Zustand gründlich mit der wäßrigen Wirkstoffaufbereitung gemischt.

Nach dem Erkalten belegt man den Nährboden mit ca. 0,1 ml Fliegeneiern und beobachtet deren Entwicklung über eine Woche.

| Wirkstoff Nr. | Mortalitätsrate [%] bei Wirkstoffkonzentrationen von | | | |
|---|---|---|---|---|
| | 50 ppm | 25 ppm | 10 ppm | 5 ppm |
| 1 | | 100 | 80 | |
| 2 | | 100 | 100 | 100 |
| 8 | | 100 | 100 | |
| 10 | | 100 | 80 | |
| 11 | | 100 | 60 | |
| 12 | | 100 | | |
| 13 | | 100 | 100 | 100 |
| 14 | | 100 | 100 | 100 |
| II | unwirksam | | | |
| III | unwirksam | | | |

## Beispiel D

### Kontaktwirkung auf Baumwollwanzen (Dysdercus intermedius)

Petrischalen von 10 cm Durchmesser werden mit 1 ml der acetonischen Wirkstofflösung ausgekleidet.

Nach Verdunsten des Lösungsmittels besetzt man die Schalen mit je 20 Larven des vorletzten Stadiums und registriert die Wirkung nach 24 Stunden.

| Wirkstoff Nr. | Mortalitätsrate [%] bei Wirkstoffmengen pro Petrischale von | | | |
|---|---|---|---|---|
| | 1 mg | 0,1 mg | 0,05 mg | 0,02 mg |
| 3 | | 100 | 100 | 80 |
| 4 | | 100 | 100 | 70 |
| 5 | | 100 | 85 | |
| II | unwirksam | | | |
| III | unwirksam | | | |

Beispiel E

Kontaktwirkung auf Blattläuse (Aphis fabae); Spritzversuch

Getopfte Bohnenpflanzen (Vicia faba) mit starken Blattlauskolonien werden in der Spritzkammer mit den wäßrigen Wirkstoffaufbereitungen tropfnaß gespritzt.

Die Auswertung erfolgt nach 24 Stunden.

| Wirkstoff Nr. | Mortalitätsrate [%] bei Wirkstoffkonzentrationen von | | | |
|---|---|---|---|---|
| | 0,1 | 0,05 | 0,02 | 0,01 |
| 1 | | 100 | 90 | |
| 4 | | 100 | 100 | 80 |
| I | unwirksam | | | |
| II | | unwirksam | | |

Beispiel F

Wirkung auf Wurzelgallennematoden (Meloidogyne incognita)

Junge Tomatenpflanzen werden in je 500 g Komposterde gepflanzt, die stark mit Wurzelgallennematoden infiziert ist.

Nach 3 Tagen erfolgt die Behandlung mit der wäßrigen Wirkstoffaufbereitung durch Gießen von 30 ml Aufbereitung.

Nach 6 bis 8 Wochen bonitiert man die Wurzeln auf Gallenbildung.

| Wirkstoff Nr. | Wirkstoffkonzentration [%] | |
|---|---|---|
| 2 | 0,025 | Grenze der Wirkung |
| 8 | 0,05 | keine Gallenbildung |
| I | 0,1 | unwirksam |
| II | 0,1 | unwirksam |

**Patentansprüche**

1. Thienylmethyl-dithiophosphorsäureester der Formel I

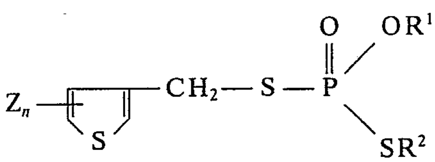

(I)

in der

R[1] Methyl oder Äthyl,
R[2] unverzweigtes oder verzweigtes Alkyl mit bis zu 5 Kohlenstoffatomen,
Z Halogen und
n 0 bis 3

bedeuten.

2. O-Äthyl-S-n-propyl-S-(2,5-dibrom-thienyl-4-methyl)-dithiophosphat.
3. O-Äthyl-S-isobutyl-S-(2,3-dichlor-thienyl-4-methyl)-dithiophosphat.

4. Verfahren zur Herstellung von Thienylmethyl-dithiophosphorsäureestern der Formel I, dadurch gekennzeichnet, daß man Thiophenderivate der Formel II

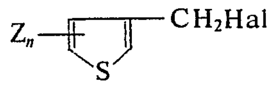

(II)

in der
Z und n die in Anspruch 1 genannten Bedeutungen haben und
Hal für Chlor oder Brom steht, mit Salzen der Formel III

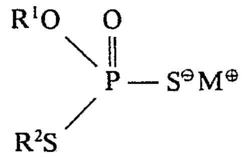

(III)

in der
$R^1$ und $R^2$ die im Anspruch 1 genannten Bedeutungen haben und
$M^{\oplus}$ für ein gegebenenfalls substituiertes Ammoniumion steht,
bei Temperaturen im Bereich zwischen 10 und 100°C in Gegenwart eines Lösungs- oder Verdünnungsmittels umsetzt.

5. Schädlingsbekämpfungsmittel, gekennzeichnet durch einen Gehalt an Thienylmethyl-dithiophosphorsäureestern der Formel I.

6. Schädlingsbekämpfungsmittel, enthaltend einen festen oder flüssigen Trägerstoff und mindestens einen Thienylmethyl-dithiophosphorsäureester der Formel I.

7. Verfahren zur Bekämpfung von Schädlingen und lästigen Gliedertieren, dadurch gekennzeichnet, daß man Thienylmethyl-dithiophosphorsäureester der Formel I auf die genannten Schädlinge bzw. deren Lebensraum einwirken läßt.

8. Verwendung von Thienylmethyl-dithiophosphorsäureester der Formel I zur Bekämpfung von schädlichen und lästigen Gliedertieren.

9. Verfahren zur Herstellung von Schädlingsbekämpfungsmitteln, dadurch gekennzeichnet, daß man Thienylmethyl-dithiophosphorsäureester der Formel I mit Streckmitteln und/oder oberflächenaktiven Mitteln mischt.

## Claims

1. Thienylmethyl-dithiophosphoric acid esters of the formula I

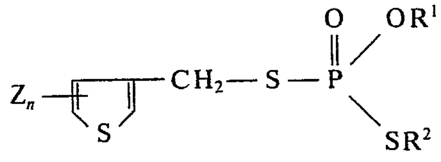

(I)

where $R^1$ denotes methyl or ethyl, $R^2$ is linear or branched alkyl of up to 5 carbon atoms, Z denotes halogen, and n denotes one of the integers 0, 1, 2 and 3.

2. O-ethyl-S-n-propyl-S-(2,5-dibromothienyl-4-methyl)-dithiophosphate.

3. O-ethyl-S-isobutyl-S-(2,3-dichlorothienyl-4-methyl)-dithiophosphate.

4. A process for the production of thienylmethyl-dithiophosphoric acid esters of the formula I, characterized in that thiophene derivatives of the formula II

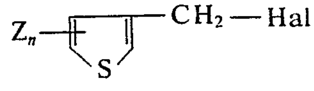

(II)

where Z and n have the meanings given in claim 1 and

Hal denotes chlorine or bromine, are reacted with salts of the formula III

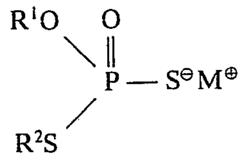

(III)

where $R^1$ and $R^2$ have the meanings given in claim 1 and $M^{\oplus}$ denotes a substituted or unsubstituted ammonium ion, at temperatures of from 10° to 100° C in the presence of a solvent or diluent.

5. A pesticide characterized by a content of thienyl-dithiophosphoric acid esters of the formula I.

6. A pesticide containing a solid or liquid carrier and at least one thienylmethyl-dithiophosphoric acid ester of the formula I.

7. A process for combating pests and troublesome articulata, characterized in that a thienylmethyl-dithiophosphoric acid ester of the formula I is allowed to act on the pests or their habitat.

8. Use of thienylmethyl-dithiophosphoric acid esters of the formula I for combating injurious and troublesome articulata.

9. A process for the production of pesticides, characterized in that a thienylmethyl-dithiophosphoric acid ester of the formula I is mixed with an extender and/or surfactant.

**Revendications**

1. Dithiophosphate de thiénylméthyle de formule

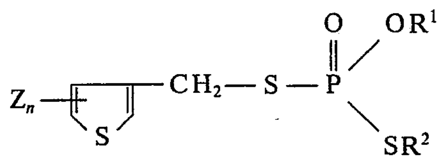

(I)

dans laquelle:

$R^1$ représente un groupe méthyle ou éthyle,
$R^2$ un alkyle ramifié ou non ayant jusqu'à 5 atomes de carbone,
Z un halogène et
n 0 à 3.

2. O-éthyl-S-n-propyl-S-(2,5-dibromo-thiényl-4-méthyl)-dithiophosphate.

3. O-éthyl-S-isobutyl-S-(2,3-dichloro-thiényl-4-méthyl)-dithiophosphate.

4. Procédé de préparation de dithiophosphate de thiénylméthyle de formule I, caractérisé par le fait qu'on fait réagir, à une température comprise entre 10 et 100° C, en présence d'un solvant ou diluant, un dérivé de thiophène de formule II

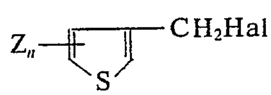

(II)

dans laquelle:
Z et n ont les significations indiquées dans la revendication 1, et
Hal représente le chlore ou le brome, avec un sel de formule III

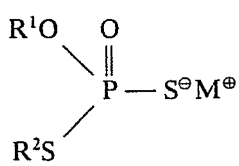

(III)

dans laquelle:
$R^1$ et $R^2$ ont les significations indiquées dans la revendication 1, et
$M^{\oplus}$ représente un ion ammonium éventuellement substitué.

5. Agent antiparasitaire caractérisé par le fait qu'il contient des dithiophosphate de thiénylméthyle de formule I.

6. Agent antiparasitaire contenant un support solide ou liquide et au moins un dithiophosphate de thiénylméthyle de formule I.

7. Procédé de lutte contre les parasites et les animaux articulés indésirables, caractérisé par le fait qu'on fait agir du dithiophosphate de thiénylméthyle de formule I sur les dits parasites ou leur biotope.

8. Utilisation de dithiophosphate de thiénylméthyle de formule I pour la lutte contre les animaux articulés nuisibles et indésirables.

9. Procédé de préparation de l'agent antiparasitaire, caractérisé par le fait qu'on mélange du dithiophosphate de thiénylméthyle de formule I avec un diluant et/ou un agent tensioactif.